(19)

Europäisches
Patentamt
European
Patent Office
Office européen
des brevets

(11)　EP 2 500 728 B1

(12)　EUROPEAN PATENT SPECIFICATION

(45) Date of publication and mention
of the grant of the patent:
**12.03.2014　Bulletin 2014/11**

(21) Application number: **11158411.6**

(22) Date of filing: **16.03.2011**

(51) Int Cl.:
***G01N 33/543*** (2006.01)

(54) **A molecular sensor using temporal discrimination**

Molekularsensor mit zeitlicher Diskriminierung

Capteur moléculaire utilisant une discrimination temporaire

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**19.09.2012　Bulletin 2012/38**

(73) Proprietor: **NXP B.V.
5656 AG Eindhoven (NL)**

(72) Inventors:
 • **Gridelet, Evelyne**
 **Redhill, Surrey RH1 1DL (GB)**
 • **Suy, Hilco**
 **Redhill, Surrey RH1 1DL (GB)**
 • **Frederix, Filip**
 **Redhill, Surrey RH1 1DL (GB)**

(74) Representative: **Hardingham, Christopher Mark
NXP B.V.
Intellectuel Property & Licensing
Red Central
60 High Street
Redhill, Surrey RH1 1SH (GB)**

(56) References cited:
**WO-A1-2009/047703**

 • **WIDDERSHOVEN F ET AL: "CMOS biosensor
platform", TECHNICAL DIGEST -
INTERNATIONAL ELECTRON DEVICES
MEETING, IEDM - 2010 IEEE INTERNATIONAL
ELECTRON DEVICES MEETING, IEDM 2010 2010
INSTITUTE OF ELECTRICAL AND ELECTRONICS
ENGINEERS INC. USA, 2010, pages 36.1.1-36.1.4,
XP002640391, DOI: DOI:10.1109/IEDM.
2010.5703484**

**Description**

<u>Field of the Invention</u>

This invention relates to molecular sensors, in particular but without limitation to biosensors.

<u>Background of the Invention</u>

It is well known to provide sensors for molecules or particles, in which the particle is bound to the surface of the sensor by some mechanism, such as a chemical bond, and thereby changes a property of the sensor. Sensing is achieved by detecting the change in this property, by for instance an electrical circuit.

As examples, a diverse range of biosensors, that is to say sensors which detects a biologically active particle or molecule, are known in which the property is typically an electrical property such as an electrical resistance or capacitance.

Discrimination between different types of particle is normally achieved in such sensors, by ensuring that only the target type, or species, of particle can properly bind to the sensor. In the case of a biosensor, this can be achieved by providing the sensing surface of the sensor with a bio-receptor particle. If the bio-receptor can be configured so it will only bind to the target biomolecule, and not to other particles, the sensing will then be specific to that target molecule. Another possible solution, where the target molecule does not uniquely associate with a bioreceptor, is to attach a label to the target molecule. In this case the bio-receptor can be made to bind specifically to the label.

Such sensors can conveniently be integrated into an electronic component, and in particular, may benefit from the billions of dollars of investment in the silicon process technology, to result in cost-effective and highly manufacturable sensors.

However, it is not always straightforward, and in some cases may not even be possible, to provide a high level of specificity for the binding of any particular target particle to the sensor.

Sensing method based on time-of-flight are known, in particular, time-of-flight mass spectroscopy (TOFMS). However, such sensor operate in the gas phase, and are thus typically inconvenient to operate, as well as involving expensive and complex equipment such as micro-channel plates linked to secondary emission multipliers. Typically, discrimination is limited to variation in mass-to-charge ratio.

Widdershoven F et Al "CMOS biosensor platform", Technical Digest - International Electron Devices Meeting IEDM - 2010 IEEE International Electron Devices Meeting, IEDM 2010, 2010 Institute of Electrical and Electronics Engineers Inc USA, 2010, discloses a detection principle, process integration and system architecture of a 90-nm CMOS spaced densely packed nano-electrodes. The publication discloses the use of such a sensor to analyse Brownian motion of nanobeads over the sensor surface.

International patent application publication number WO2009/047703 discloses a sensor, a sensor array and a method of operating a sensor, the sensor comprising an electrode and having a sensor active region covering the electrode and being sensitive for particles. A first switch element is operable to bring the electrode to a first electric potential when the first switch element is closed; a second switch element is operable to bring the electrode to a second electric potential when the second switch element is closed. A detector is adapted to detect particles based on a change of the electric properties of the sensor.

It would therefore be desirable to provide an inexpensive sensor for a particle and in particular for a biomolecule, which does not rely on the specificity of a binding event for the discrimination between the target molecule and other, non-target, molecules.

<u>Summary of the invention</u>

It is an object if of the present invention to provide such a sensor.

According to the present invention there is provided a sensor device as defined in claim 1. The sensor device may alternatively be called a molecular sensor, since it is adapted to sense particles being either single particles and groups of particles.

It will be appreciated that, in contrast to other sensing methods, particularly using functionalised beads such as fluorescence , a sensor device according to the invention advantageously may operate in real-time, rather than requiring post-process analysis such as is the case for conventional electrophoresis.

In embodiments the arrangement of a plurality of sensors comprises either at least 1000 sensors or at least 10,000 sensors or at least 65,000 sensors. The sensor device thus may be massively parallel.

In embodiments, the sensors are arranged in an array on a major surface of the sensor, each sensor occupying a surface area of no more than 100 $\mu m^2$ of the major surface.

In embodiments, the sensor device configured to discriminate in time between a first group of binding events of particles to respective nano-electrodes and a second group of binding events of particles to respective nano-electrodes, where the first group of binding events and the second group of binding events are separated by more than 1ms or more than 100ms or more than 10s. It will be appreciated that there need not necessarily be a separation between the groups, particularly when the groups include significant temporal spread - discrimination may be based on the identification of separate peaks even when the groups' distributions overlap.

In embodiments at least one of the first group of binding events and the second group of binding events involves binding no more than 1000 particles, no more than 50 particles, or even only a single particle. In the latter embodiment, the sensor device may be arranged to count individual binding events. It will be appreciated that reliable time-discrimination may be achievable only with a plurality of binding events, since the timing of any single binding event may be distorted - or even prevented - by the thermal motion of individual particles even where the thermal energy is, on average, significantly less than a field energy driving the discrimination.

Preferred embodiments comprise a biosensor configured for sensing biologically active molecules or particles.

In embodiments, the nano-electrode is configured to bind a biologically active particle by means of at least one of a self-assembled monolayer and a bio-receptor particle.

In embodiments, sensor device further comprises transport means for transporting a plurality of species of particle to the nano-electrodes from a reservoir, wherein different species of particle take respectively different times to traverse the transport means from the reservoir to the nano-electrodes.

In embodiments, the transport means is configured such that different species of particle take respectively different times to traverse the transport means from the reservoir to the nano-electrodes due to one of the group of phenomena comprising elutriation (also known as gravitational sedimentation), electrophoresis, magnetophoresis, electromagneto-phoresis, thermophoresis, and chromatography.

It will be appreciated that some embodiments of the invention do not require immobilize probe biomolecules on the sensor. This is often carried out be means of self-assembled monolayers, which are attached to the electrodes, Such embodiments thus strongly simplify the processing of the biosensor and increase its shelf life.

Furthermore, since in embodiments which do not require labels, the sensing is generally not dependant on particular probe particles or bioreceptors, the expense, and inconvenience of selecting or preparing the bio-receptor, or multiplexing them on the sensor, may be avoided.

These and other aspects of the invention will be apparent from, and elucidated with reference to, the embodiments described hereinafter.

Brief description of Drawings

Embodiments of the invention will be described, by way of example only, with reference to the drawings, in which

Figure 1 illustrates a sensing array, where regions of the array are specific to different particles;
Figure 2 is a plan view of a sensing array as shown in figure 1;
Figure 3 is a schematic cross-section through part of a sensor array;
figure 4 illustrates schematically embodiments of the invention; and
figure 5 shows an exemplary temporal response from a sensor array according to embodiments of the invention, and
figure 6 illustrate the functionalization of beads, for labelled detection of particles.

It should be noted that the Figures are diagrammatic and not drawn to scale. Relative dimensions and proportions of parts of these Figures have been shown exaggerated or reduced in size, for the sake of clarity and convenience in the drawings. The same reference signs are generally used to refer to corresponding or similar feature in modified and different embodiments

Detailed description of embodiments

Recently, the present Applicant has developed a sensor, having particular applications as a biosensor, and which provides for massively parallel sensing. By massively parallel is meant a sensor which can detect at least 1,000 particles or particles, either individually or in groups. Massively parallel sensing is a sub-set of multiplexed, or multi-analyte, sensing; a multiplexed sensor can detect a plurality of molecules or particles - typically at least 5 molecules or particles, either individually or in groups. This sensor, which is disclosed in co-pending patent application publication number W02008/132656, relies on impedance sensing, and is based on the change in impedance when a particle, which is typically a biomolecule, binds to an electrode. By providing the sensor electrode as a nano-electrode (that is to say, the electrode occupies an area of less than 1 $\mu m^2$ on the surface of the sensor, and is thus an electrode on the scale of nanometres, rather than, for instance, micrometers, or millimetres), the biosensor can comprise an array of many tens of thousands of individual sensors. In an example prototype embodiment, the sensor comprises 65,200 individual nano-electrodes.

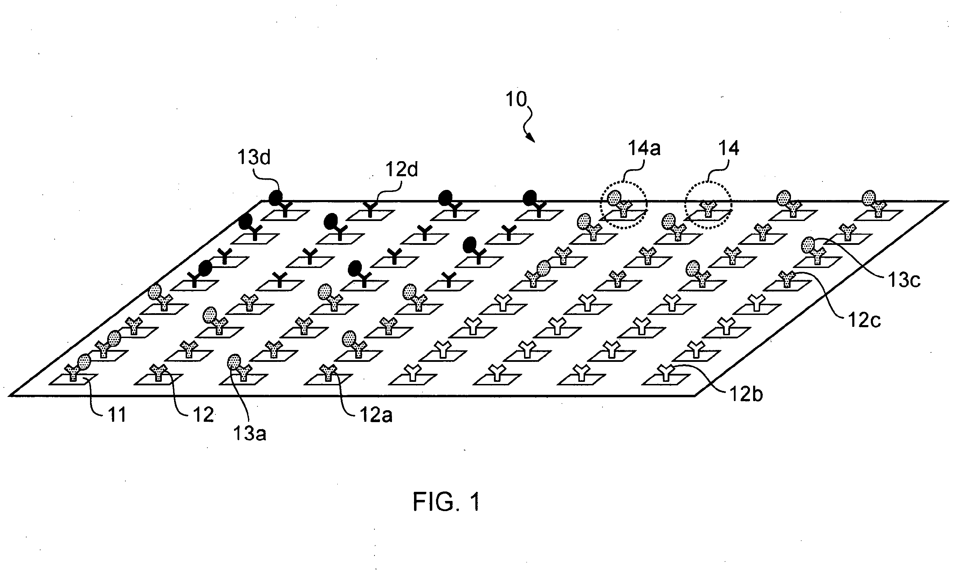

The nano-electrodes in different regions of the array may be sensitised to different target particles or biomolecules. This is shown schematically in Figure 1, which show a sensor array 10, having an array of individual sensors each having a nano-electrode 11. In the exemplary figure shown, the array is an 8 x 8 array. To each nano-electrode is attached a receptor molecule 12, which is typically a bio-receptor. In different regions of the array, different bioreceptors 12a, 12b, 12c and 12d are attached or bonded to the nano-electrodes. In the exemplary figure, the array comprises four regions, each of which has a 4x4 sub-array of sensors, and thus a 4x4 sub-array of nano-electrodes. The different bioreceptors 12a, 12b, 12c and 12d are sensitive to respective different biomolecules 13a, 13b, 13c (not shown) and 13d. Each different biomolecule can bind only with the appropriate bio-receptor, and not to any other bio-receptor. The nano-electrode 11 together with bioreceptor 12, and with or without a bound biomolecule forms a nano-electrode structure 14, 14a.

When a sample analyte, which contains one or more of the species of biomolecules 13a, 13b, 13c and 13d is passed over the sensor array 10, the biomolecules bind to the respective biosensors. In the examples shown, the analyte includes molecules 13a 13c and 13d but no molecules of type 13b, and thus no biomolecules bind to this area of the array 10.

The sensor operates by detecting a change in the capacitance formed by the nano-electrode structure, analyte and counter electrode (not shown), when the biomolecule binds to the bioreceptor, thus changing the nano-electrode structure 14 to modified nano-electrode structure 14a.

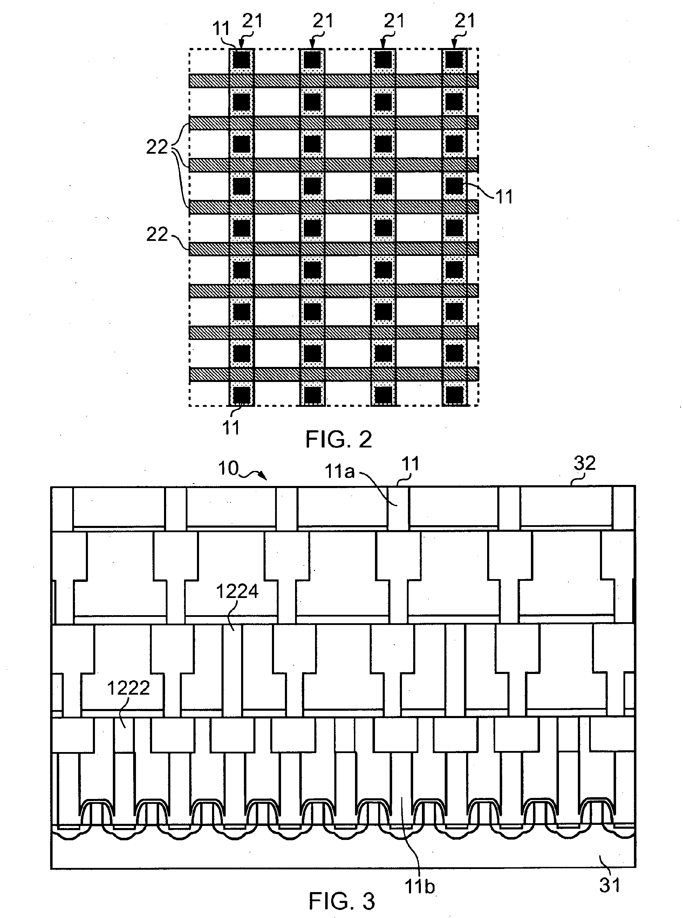

Figure 2 shows a plan view of part of a biosensor array. As shown, the sensors are conveniently arranged in a Cartesian or X-Y grid configuration, such that individual nano-electrodes 11 corresponding to individual sensors can be addressed by means of rows 22 and columns 21.

Figure 3 shows a schematic cross-section of part of an array 10 of sensors. The nano-electrodes 11 extend from the surface of the sensor array into the bulk of a semiconductor 31, by means of known Damascene structures. The top section 11 a of the nano-electrodes, on the surface 32 of the sensor array is thus environmentally isolated from the bottom section 11 b of the nano-electrodes. The bottom section 11b can form part of an electronic circuit, as is known and described in co-pending patent application publication WO2008/132656.

The electronic circuit described in the above reference co-pending patent application responds quickly. The sensing may thus be considered to be "in real time", and does not rely on either extensive post-processing of data, or "before-and-after" measurements (that is to say measurements made before and after the binding event, with a difference indicating a binding event). This is because a large part of the data treatment is made on the detection chip, which is possible because the biosensor is provided directly on top of standard CMOS; real-time monitoring of 65000 individual electrodes is thus enabled. Further, it is possible to determine with a high degree of accuracy, the moment at which the binding of the biomolecule to the bioreceptor occurs. It is thus possible to distinguish between groups of binding event, which occur at different moments. It will be understood, that even in the absence of a particularly fast response of the electronic circuit, provided the response time is consistent, then the *relative* time at which the binding event occurs will can still be determined.

Moreover, the electronic circuit is also to particularly sensitive, such that it is possible to detect a small number of binding events. With the known circuitry it is possible to detect as few as 100 or even 10 binding events. With appropriate optimisation of the circuit, it may be possible to detect even a single binding event.

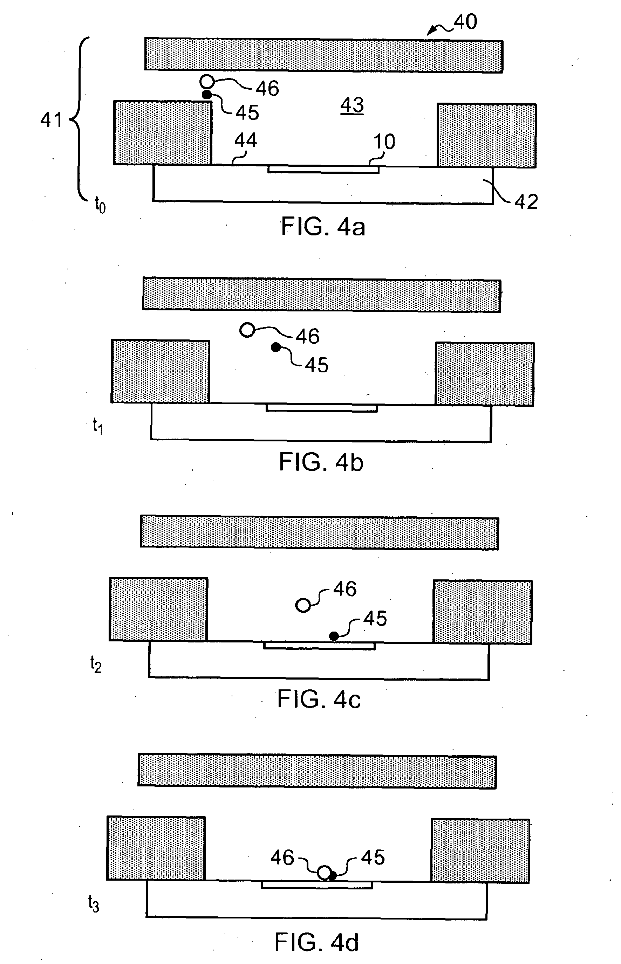

Figure 4 shows schematically, at Fig 4a, Fig 4b, Fig 4c and Fig 4d, a sensor array arrangement 40 according to embodiments of the invention at different moments to, $t_1$, $t_2$ and $t_3$ after an analyte is introduced into the sensor array arrangement 40 comprises a microfluidic part 41, together with a sensor array part 42. The microfluidic part 41 comprises a microfluidic channel 43. Over part of its length, the microfluidic channel is in contact with sensor array part 42, and specifically with sensitive area 10 of the sensor array. For simplicity, the sensitive area is shown as a single block; however, the skilled person will appreciate that the sensitive area 10 is comprised of a plurality of individual sensors, each of which has a nano-electrode 11 which extends from the surface of the sensor, which surface is in contact with the year microfluidic channel 43, into the body of the sensor array part 42. The sensor array part 42 may conveniently be fabricated as an electronic component, and is typically based on a silicon chip.

As shown at figure 4a, at time to, an analyte comprising molecules types or species 45 and 46, is introduced at the start of the microfluidic channel 43. Typcially, the molecule species 45 and 46 will be different species. The presence of, or even the concentration of, one of the species 45 and 46 may be known, and act as a reference species. The analyte may contain two different species of interest, and which may bind to the sensitive area 10 of the sensor array, or a greater number of different species.

Different molecule types or species 45 and 46 may traverse the microfluidic channel 43 at different rates (based on a difference in a property such as their size), as will be explained in more detail hereinbelow, and thus at a later time $t_1$ molecule 45 has travelled further than molecule 46, as shown in figure 4b.

Figure 4c shows the sensor array arrangement 40 at a yet later time $t_2$. By this time, the faster travelling molecule 45 has reached the sensitive are 10 of the sensor array part 42; however, the slower moving molecule 46 is still in flight.

By in flight is meant that it has not reached the sensitive area and is thus still in transit. The sensor may detect the binding of an individual molecule 45 to one of the nano-electrode structures forming part of the sensor array. However, even if the sensor is not sufficiently sensitive to detect the binding of an individual molecule 45, it will detect the group of binding events of a plurality of molecules 45, as will be discussed further below.

Finally, as shown at figure 4d, the other, slower moving molecule 46 arrives at the sensitive area 10 of the sensor array part 42, and binds to a nano-electrode. At this time, $t_3$, the sensor will detect another binding event. Thus, the sensor is able to discriminate between the different types of molecules 45 and 46, based on their different time of flight in the microfluidic channel 43.

It will be noted that in this example, only a single molecule of each type 45 and 46 is shown; to successfully discriminate between the two molecules, the electronics of the sensor must be sufficiently sensitive to be able to detect a single binding event. However, in general, or there will be many hundreds , thousands, millions, or even billions, of molecules of each individual type 45 and 46 which reach the sensitive area, depending on the concentration of each type of molecule within the analyte. Provided that the time of flight of any individual type of molecule, 45 or 46, is relatively constant, as will be described in more detail below, it can be expected that most of the molecules of type 45 will reach the sensitive area of the sensor array, that is broadly the same time $t_2$, and equivocally most of the molecules of the other type 45 will reach the sensitive area of the sensor array at broadly the same time, $t_3$. Thus the sensor need only be able to detect a group of binding events corresponding to the lower of the expected number of molecules 45 and 46, which reach the sensitive area of the sensor array.

As shown in figure 4, the length of the microfluidic channel 43 is small relative to the size of the sensitive area 10 of the sensor array 42. However, the skilled person will appreciate that this diagram is highly schematic, and in the most practical configurations, the microfluidic channel will be significantly longer than the length of the sensitive area, in order to allow for an appropriate level of discrimination in time of flight between different types of molecule. In preferred practical arrangements, it will be arranged that the time taken for molecules to travel between the furthest separated individual nano-electrodes of the sensitive area of the sensor array, is smaller than the expected difference in time, between the two types of molecules reaching the sensitive area, achieved by the time of flight discrimination

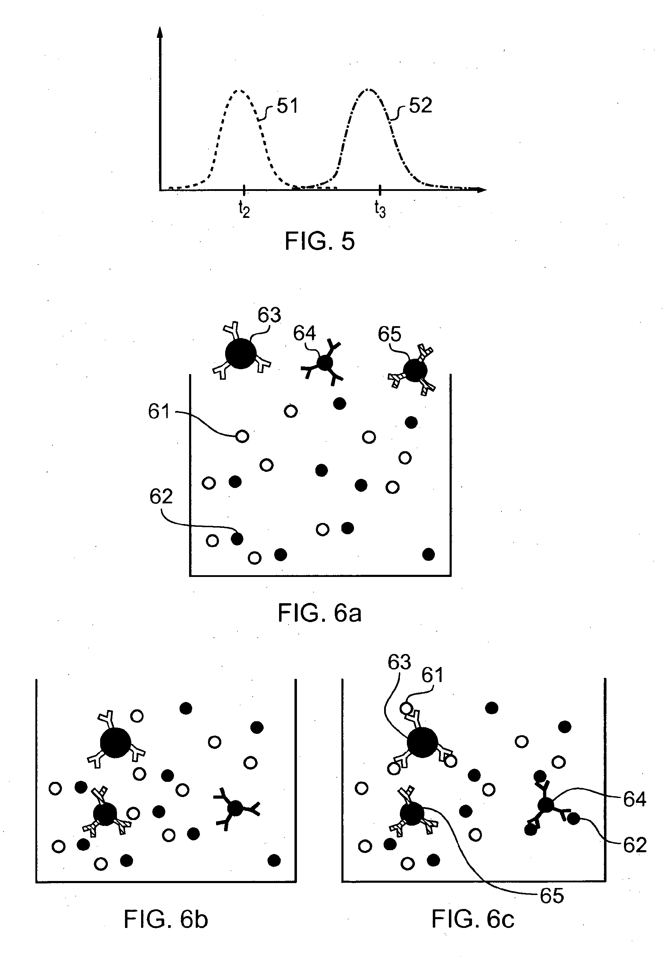

Figure 5 is a graph showing, schematically, a representation of an output signal from the electronics of the sensor array. The signal (on the ordinate or y-axis) represents the differential of the the number of electrodes where a detection event has occurred, plotted against time (on the abscess or x-axis). There is a first change in capacitance, which occurs around time $t_2$ and corresponds to the arrival of the first type or species of molecule 45 at the sensitive area. This is shown by first part of the curve, 51 (drawn as the dashed line figure 5). Around a second, later, time $t_3$ there is a further peak in the differential signal, corresponding to in a further increase in capacitance in the sensor array due to the arrival of the second type or species of molecule 46.

The spread in both the first and the second peaks is due to the spread in binding moments of each type of molecule. This results firstly from the fact that individual models will bind at individual sites which may be at different distances from the start of the microfluidic channel 43 where the analyte is introduced, and thus the individual molecules has different distances to travel before binding. Secondly, individual molecules do not traverse the microfluidic channel at exactly the same speed, so there will be a spread in the moment of arrival of individual molecules, due to nor more diffusion mechanisms which will be well understood by the skilled person. In other embodiments, the microfluidic channel is replaced by a gel or chromatographic column which changes the speed towards the sensor surface. This can make the effects more clearly visible and although it may require some sample preparation.

The relative size of the peaks in the signal, is indicative of the number of molecules binding. Thus the signal may be used to provide an indication of the relative concentration, or even under some circumstances such as with appropriate calibration, the absolute concentration, or the species of molecules. This may be the case, even in embodiments in which the sensor is insufficiently sensitive to detect individual binding events. Of course, in embodiments in which the sensor is sufficiently sensitive to detect individual binding events, it may be possible to determine an indication of the relative or absolute concentrations of molecules 45 or 46 by means of digitally counting the binding events, as an alternate to measuring an analog signal such as the area under the peak of the signal 51, 52.

Significant to embodiments of the invention is thus the fact that it is possible to discriminate between the time of arrival at the sensitive area, for different types of molecule. This function may be carried out in a microfluidic part, which may be as shown in figure 4 integrated together with the sensor array. However, the microfluidic part used to effect the discrimination between different types of molecule may be physically separate or spaced apart from the sensitive area of the sensor array, or the discrimination may be effected in a separate component or subunit such as gels or chromatographic columns.

The discrimination may be achieved by one of several different mechanisms, or a combination of them; exemplary such mechanisms which will now be considered:

A first method of discrimination is chromatography: Chromatography is well-known to the skilled person. A liquid analyte with molecules for detection is be passed first through a chromatograph before reaching the sensitive area.

The molecules with the shorter retention time will come out earlier from the chromatograph and arrive at the sensitive area earlier than the molecules with a longer retention time.

Other mechanisms involve the application of a field. The field may be an gravitational field, electrical field, magnetic field, in the cases respective of the discrimination being by elutriation (which may also be referred to as gravitational sedimentation), electrophoresis and magnetophoresis. The appropriate field is applied in order to create a motion in the direction towards the sensitive area. The field may be applied in the microfluidic channel, or upstream of this channel. Molecules will move according to their size and their response in the field (mass, charge, magnetic susceptibility). In general, a more charged molecule will move faster than a less charged molecule, arrive at the sensitive area of the sensor before the less charged molecule and be detected before.

Under the approximation that the molecule is spherical, then, constant drift speed v of a spherical molecule in motion in a given field is found by equating the frictional force $6\Pi\eta av$, where $\eta$ is the viscosity and a the radius of the molecule, with the driving force F

$$F=6\pi\eta av \qquad (1)$$

so that the drift speed depends on the applied force and on the size of the molecule

$$v = F/6\pi\eta a \qquad (2)$$

However, if the thermal energy = kT is higher than the work of applied force, the effect of the applied force will be negligible and the thermal motion will dominate.

In the case of an electric field, the work associated with the motion of a particle in an electric field is

$$W = z_e Eh \qquad (3)$$

with $z_e$ being the charge of the particle, E the electric field, and h the distance. The work of the electric force can be orders of magnitude higher than the thermal energy. It can be tuned by changing the pH of the solution and thus the charge of the molecule, or tuning the electric field.

It is known that electrophoresis works better in gel than in simple aqueous medium. In this case, the mobility of a linear biomolecule like DNA is inversely proportional to its molecular length, and it is thus readily feasible to discriminate between different species of molecules such as different DNA strands.

In the case of a gravitational field, the work associated with the motion of a particle in the gravitational field is

$$W = mgh = 4/3 \ \pi a^3 (\rho - \rho_{liquid})gh \qquad (4)$$

Where m is the mass of the particle, p its density, $\rho_{liquid}$ the liquid density, $g = 9.81$ m/s$^2$, h the vertical distance.

For a 10nm molecule (which is the typical size of a biomolecule of most interest), the thermal energy is 4E-21 J while the gravitational work for a 0.3mm distance is 5E-25J. Thus the thermal motion will dominate and so a gravitational field is not sufficient to actuate unlabelled biomolecules.

Labelling biomolecules is well-known to the skilled person. Typically, labels are nanobeads functionalized with chemical groups or biomolecules. They have to be chosen to bind to the biomolecules of interest and be premixed with them, as will now be described with reference to Figure 6.

At Figure 6(a) is shown a solution with two types of biomolecules 61, 62 and beads 63, 64, 65 functionalized with probes corresponding to the biomolecules suspected to be present in the solution.

At Figure 6(b) the beads are mixed with the biomolecules.

At Figure 6(c) it is shown that the beads capture the probe biomolecules corresponding to their functionalization. Shown is bead 63 which binds with (or is functionalised by) biomolecule 61, and bead 64 which binds with (or is functionalised by) biomolecule 62; there are no biomolecules which functionalise the third bead type, 65. The unbound beads are filtered out., according to known techniques - for example their functional group can be bound to other kind of biomolecules, or by chromatography.

For 500nm beads, the thermal energy is 4E-21 J while the gravitational work for a 0.3mm distance is 4E-19J. It means that the gravitational motion will dominate.

The drift velocity is

$$v = mg/6\pi\eta a = 4/3\ \pi a^3(\rho-\rho_{liquid})g/6\pi\eta a = 2/9\ a^2(\rho-\rho_{liquid})g/\eta \quad (5)$$

With typical values of the parameters, the difference in time for a 500nm and for 400nm beads is about 10 minutes, which is easily measurable.

Magnetic fields can be applied, at least with labelled molecules: Functionalized nano-beads have often a magnetic core and can be actuated with a magnetic field. In that case, the force is

$$F_{mag} = \frac{(\chi_2 - \chi_1)V.B.(\nabla B)}{\mu_0} \qquad (6)$$

Where $\chi_2$ is the volume magnetic susceptibility of the magnetic particle, $\chi_1$ is the volume magnetic susceptibility of the surrounding medium, p is the magnetic susceptibility of free space, V is the magnetisable volume of the bead, and B is the magnetic flux. That is to say, the force depends on the magnetic susceptibility of the beads and of their size.

The size of the beads and the magnetic field are preferably chosen in order to make the discrimination between several beads possible.

Moreover, electrical fields can be applied with labelled molecules, in addition to with unlabelled molecules as discussed above: the work of the electric force can be orders of magnitude higher than the thermal energy. It can be tuned by changing the pH of the solution and thus the charge of the biomolecule, or tuning the electric field.

The drift velocity is

$$v = zeE/6\pi\eta a \qquad (7)$$

which means that the motion of the particle will depend on the ratio z/a between their charge z and radius a. Since for functionalized beads, the density of charge per unit of surface is constant (z is proportional to $a^2$), it means that the velocity is actually directly proportional to a, the radius of the bead. With typical values of the parameters, the difference in time for 500nm and for 400nm beads is about 5 minutes, which is easily measurable.

In summary, then, from one viewpoint a sensor device is disclosed, which depends on discrimination in time between groups of binding events of target molecules to nano-electrodes. The target molecules may be in the liquid phase or in suspension. The nano-electrodes form part of a sensor arrangement having a plurality of sensors. The sensor device is arranged such that different species of target molecules arrive at the nano-electrodes at different times, using techniques such as chromatography or application of a field such as an electric, magnetic, or gravitational field. The molecules may be labelled or unlabeled. The invention is particularly suited, but not limited, to sensing biomolecules.

From reading the present disclosure, other variations and modifications will be apparent to the skilled person. Such variations and modifications may involve equivalent and other features which are already known in the art of molecular sensors, and which may be used instead of, or in addition to, features already described herein.

**Claims**

1. A sensor device comprising a biosensor configured for sensing biologically active particles, the sensor device, comprising:

   an arrangement of a plurality (10) of sensors for sensing an analyte which is in at least one of liquid phase or a suspension or a gel, each sensor comprising a nano-electrode (11) and being configured to sense the presence of a particle (13a, 13b, 13c, 13d) localised to or bound to the nano-electrode,
   wherein the sensor is configured to discriminate in time the binding of particles to respective nano-electrodes; and
   transport means for transporting a plurality of species of particle to the nano-electrodes from a reservoir, wherein different species of particle take respectively different times to traverse the transport means from the reservoir to the nano-electrodes .

**2.** A sensor device as claimed in claim 1, wherein the transport means is configured such that different species of particle take respectively different times to traverse the transport means from the reservoir to the nano-electrodes due to one of the group of phenomena comprising elutriation, electrophoresis, magnetophoresis, electromagnetophoresis, thermophoresis, and chromatography.

**3.** A sensor device as claimed in claim 1 or 2, wherein the arrangement of a plurality of sensors comprises either at least 1000 sensors or at least 10,000 sensors or at least 65,000 sensors.

**4.** A sensor device as claimed in any preceding claim, wherein the sensors are arranged in an array on a major surface of the sensor, each sensor occupying a surface area of no more than 100 $\mu$m$^2$ of the major surface.

**5.** A sensor device as claimed in any preceding claim configured to discriminate in time between a first group of binding events of particles to respective nano-electrodes and a second group of binding events of particles to respective nano-electrodes, where the first group of binding events and the second group of binding events are separated by more than 1 ms.

**6.** A sensor device as claimed in claim 5 wherein the first group of binding events and second group of binding events are separated by more than 100ms.

**7.** A sensor device as claimed in claim 5 wherein the first group of binding events and second group of binding events are separated by more than 10s.

**8.** A sensor device as claimed in any of claims 5 to 7, wherein at least one of the first group of binding events and the second group of binding events involves binding no more than 1000 particles.

**9.** A sensor device as claimed in claim 8, wherein at least one of the first group of binding events and the second group of binding events involves binding no more than 50 particles.

**10.** A sensor device as claimed in claim 8, wherein at least one of the first group of binding events and the second group of binding events involves binding only a single particle.

**11.** A sensor device as claimed in any preceding claim, wherein the nano-electrode is configured to bind a biologically active particle by means of at least one of a self-assembled monolayer and a bio-receptor particle (12a, 12b, 12c, 12d).

**Patentansprüche**

**1.** Eine Sensor Vorrichtung aufweisend einen Biosensor, welcher konfiguriert ist zum Abtasten von biologisch aktiven Teilchen, wobei die Sensor Vorrichtung aufweist:

eine Anordnung von einer Mehrzahl (10) von Sensoren zum Abtasten eines Analyten, welcher vorliegt in zumindest einem von einer flüssigen Phase oder einer Suspension oder einem Gel, wobei jeder Sensor eine Nano-Elektrode (11) aufweist und konfiguriert ist, um die Anwesenheit eines Teilchens (13a, 13b, 13c, 13d) abzutasten, welches an der Nano-Elektrode lokalisiert oder gebunden ist,
wobei der Sensor konfiguriert ist, um zu unterscheiden nach der Zeit der Bindung der Teilchen an jeweilige Nano-Elektroden; und
Transportmittel zum Transportieren einer Mehrzahl von Teilchenarten zu den Nano-Elektroden von einem Behälter, wobei verschiedene Teilchenarten jeweils verschiedene Zeiten benötigen, um das Transportmittel von dem Behälter zu den Nano-Elektroden zu durchlaufen.

**2.** Eine Sensor Vorrichtung gemäß Anspruch 1, wobei das Transportmittel so konfiguriert ist, dass verschiedene Teilchenarten jeweils verschiedene Zeiten benötigen, um das Transportmittel von dem Reservoir zu den Nano-Elektroden zu durchlaufen, aufgrund eines der Gruppe von Phänomenen, welche aufweisen Auswaschen, Elektrophorese, Magnetophorese, Elektro-Magnetophorese, Thermophorese und Chromatographie.

**3.** Eine Sensor Vorrichtung gemäß Anspruch 1 oder 2, wobei die Anordnung einer Mehrzahl von Sensoren aufweist, entweder zumindest 1000 Sensoren oder zumindest 10 000 Sensoren oder zumindest 65 000 Sensoren.

**4.** Eine Sensor Vorrichtung gemäß irgendeinem vorangehenden Anspruch, wobei die Sensoren in einem Array auf einer Hauptoberfläche des Sensors angeordnet sind, wobei jeder Sensor eine Oberfläche besetzt von nicht mehr als 100 $\mu m^2$ der Hauptoberfläche.

**5.** Eine Sensor Vorrichtung gemäß irgendeinem vorangehenden Anspruch, welche konfiguriert ist, um zu unterscheiden nach der Zeit zwischen einer ersten Gruppe von Bindungsereignissen von Teilchen an jeweilige Nano-Elektroden und einer zweiten Gruppe von Bindungsereignissen von Teilchen an jeweilige Nano-Elektroden, wo die erste Gruppe von Bindungsereignissen und die zweite Gruppe von Bindungsereignissen durch mehr als 1 ms getrennt sind.

**6.** Eine Sensor Vorrichtung gemäß Anspruch 5, wobei die erste Gruppe von Bindungsereignissen und die zweite Gruppe von Bindungsereignissen durch mehr als 100 ms getrennt sind.

**7.** Eine Sensor Vorrichtung gemäß Anspruch 5, wobei die erste Gruppe von Bindungsereignissen und die zweite Gruppe von Bindungsereignissen durch mehr als 10 s getrennt sind.

**8.** Eine Sensor Vorrichtung gemäß irgendeinem der Ansprüche 5 bis 7, wobei zumindest eines von der ersten Gruppe von Bindungsereignissen und der zweiten Gruppe von Bindungsereignissen das Binden von nicht mehr als 1000 Teilchen beinhaltet.

**9.** Eine Sensor Vorrichtung gemäß Anspruch 8, wobei zumindest eines von der ersten Gruppe von Bindungsereignissen und der zweiten Gruppe von Bindungsereignissen das Binden von nicht mehr als 50 Teilchen beinhaltet.

**10.** Eine Sensor Vorrichtung gemäß Anspruch 8, wobei zumindest eines aus der ersten Gruppe von Bindungsereignissen und der zweiten Gruppe von Bindungsereignissen das Binden nur eines einzelnen Partikels beinhaltet.

**11.** Eine Sensor Vorrichtung gemäß irgendeinem vorangehenden Anspruch, wobei die Nano-Elektrode konfiguriert ist, um ein biologisch aktives Teilchen zu binden mittels zumindest einem von einer selbstorganisierten Einzelschicht und einem Bio-Rezeptor Teilchen (12a, 12b, 12c, 12d).

**Revendications**

**1.** Dispositif de détection comprenant un biocapteur configuré pour détecter des particules biologiquement actives, le dispositif de détection comprenant :

un agencement d'une pluralité (10) de capteurs pour détecter un analyte qui se trouve dans une phase liquide et/ou une suspension et/ou un gel, chaque capteur comprenant une nanoélectrode (11) et étant configuré pour détecter la présence d'une particule (13a, 13b, 13c, 13d) localisée sur ou liée à la nanoélectrode, le capteur étant configuré pour discriminer temporellement la liaison de particules à des nanoélectrodes respectives ; et
un moyen de transport pour transporter une pluralité d'espèces de particules jusqu'aux nanoélectrodes depuis un réservoir, différentes espèces de particules prenant respectivement des temps différents pour traverser le moyen de transport du réservoir aux nanoélectrodes.

**2.** Dispositif de détection selon la revendication 1, dans lequel le moyen de transport est configuré de telle sorte que différentes espèces de particules prennent respectivement des temps différents pour traverser le moyen de transport du réservoir aux nanoélectrodes en raison d'un phénomène du groupe de phénomènes comprenant l'élutriation, l'électrophorèse, la magnétophorèse, l'électromagnétophorèse, la thermophorèse et la chromatographie.

**3.** Dispositif de détection selon la revendication 1 ou 2, dans lequel l'agencement d'une pluralité de capteurs comprend au moins 1000 capteurs ou au moins 10 000 capteurs ou au moins 65 000 capteurs.

**4.** Dispositif de détection selon l'une quelconque des revendications précédentes, dans lequel les capteurs sont disposés en un réseau sur une surface principale du capteur, chaque capteur occupant une superficie inférieure ou égale à 100 $\mu m^2$ de la surface principale.

**5.** Dispositif de détection selon l'une quelconque des revendications précédentes configuré pour discriminer temporellement un premier groupe d'événements de liaison de particules à des nanoélectrodes respectives et un deuxième groupe d'événements de liaison de particules à des nanoélectrodes respectives, le premier groupe d'événements de liaison et le deuxième groupe d'événements de liaison étant séparés par plus de 1 ms.

**6.** Dispositif de détection selon la revendication 5, dans lequel le premier groupe d'événements de liaison et le deuxième groupe d'événements de liaison sont séparés par plus de 100 ms.

**7.** Dispositif de détection selon la revendication 5 dans lequel le premier groupe d'événements de liaison et le deuxième groupe d'événements de liaison sont séparés par plus de 10 s.

**8.** Dispositif de détection selon l'une quelconque des revendications 5 à 7, dans lequel le premier groupe d'événements de liaison et/ou le deuxième groupe d'événements de liaison impliquent la liaison de pas plus de 1000 particules.

**9.** Dispositif de détection selon la revendication 8, dans lequel le premier groupe d'événements de liaison et/ou le deuxième groupe d'événements de liaison impliquent la liaison de pas plus de 50 particules.

**10.** Dispositif de détection selon la revendication 8, dans lequel le premier groupe d'événements de liaison et/ou le deuxième groupe d'événements de liaison impliquent la liaison d'une seule particule.

**11.** Dispositif de détection selon l'une quelconque des revendications précédentes, dans lequel la nanoélectrode est configurée pour lier une particule biologiquement active au moyen d'une monocouche auto-assemblée et/ou d'une particule bioréceptrice (12a, 12b, 12c, 12d).

FIG. 1

FIG. 2

FIG. 3

FIG. 4a

FIG. 4b

FIG. 4c

FIG. 4d

FIG. 5

FIG. 6a

FIG. 6b

FIG. 6c

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009047703 A

- WO 2008132656 A

**Non-patent literature cited in the description**

- CMOS biosensor platform. **WIDDERSHOVEN F et al.** Technical Digest - International Electron Devices Meeting IEDM - 2010 IEEE International Electron Devices Meeting, IEDM 2010. Institute of Electrical and Electronics Engineers Inc, 2010